# EUROPÄISCHE PATENTSCHRIFT

(11) **EP 3 768 184 B1**
(45) Veröffentlichungstag und Bekanntmachung des Hinweises auf die Patenterteilung: **12.08.2026**
(21) Anmeldenummer: 19715832.2
(22) Anmeldetag: 20.03.2019
(51) Int. Cl.: A61B 18/12

(54) **ELEKTROCHIRURGIESYSTEM**
ELECTROSURGICAL SYSTEM
SYSTÈME ÉLECTROCHIRURGICAL

(30) Priorität: 21.03.2018 DE 102018106737
(43) Veröffentlichungstag der Anmeldung: 27.01.2021
(73) Patentinhaber: Olympus Winter & Ibe GmbH, 22045 Hamburg (DE)
(72) Erfinder: KRÜGER, Jens, 15732 Eichwalde (DE)
(74) Vertreter: Eisenführ Speiser
(86) Internationale Anmeldenummer: PCT/EP2019/056953
(87) Internationale Veröffentlichungsnummer: WO 2019/180078

(56) Entgegenhaltungen:
- DE-A1- 102009 018 100
- DE-A1- 102009 018 107
- DE-U1- 202011 000 441
- GB-A- 2 450 679
- US-A1- 2011 125 149

## Beschreibung

Die Erfindung betrifft ein Elektrochirurgiesystem, welches einen Generator und wenigstens zwei mit dem Generator verbundene Aktivierungsschalter und wenigstens ein mit dem Generator verbundenes Elektrochirurgieinstrument umfasst.

Derartige Elektrochirurgiesysteme sind bekannt, um beispielsweise mithilfe des Elektrochirurgieinstruments Körpergewebe zu koagulieren oder zu schneiden oder beides. Der Generator ist in diesem Falle typischerweise ein Hochfrequenzgenerator, der ausgebildet ist, an das Elektrochirurgieinstrument hochfrequente Wechselspannung abzugeben. Das Elektrochirurgieinstrument besitzt typischerweise eine Elektrodensonde, die typischerweise einen oder mehrere Elektrodenpole aufweist und demensprechend monopolar oder bipolar ausgebildet sein kann. Im Falle eines bipolaren Elektrochirurgieinstruments besitzt die Sonde zwei Elektrodenpole in einem Abstand zueinander. Ein monopolares Instrument besitzt eine Sonde mit nur einem Elektrodenpol. Monopolare Elektrochirurgieinstrumente werden typischerweise in Verbindung mit einer großflächigen Patch-Elektrode verwendet, die im Einsatzfall einen Körper eines Patienten kontaktiert.

An einen Generator eines Elektrochirurgiesystems können häufig mehrere gleiche oder unterschiedliche Instrumente angeschlossen werden. Ebenso bieten Generatoren von Elektrochirurgiesystemen häufig die Möglichkeit, ein jeweiliges Elektrochirurgieinstrument in wenigstens zwei verschiedenen Betriebsmodi zu betreiben. Ein Betriebsmodus kann beispielsweise dem Koagulieren von Körpergewebe dienen, während ein anderer Betriebsmodus dem Schneiden von Körpergewebe mithilfe eines Lichtbogens dienen kann.

Um ein jeweiliges Elektrochirurgieinstrument oder einen jeweiligen Betriebsmodus oder ein jeweiliges Elektrochirurgieinstrument in einem bestimmten Betriebsmodus zu aktivieren, sind Schalter vorgesehen, die von einem Arzt während der Behandlung betätigt werden können und deren Betätigen das Aktivieren eines jeweiligen Betriebsmodus oder eines jeweiligen Elektrochirurgieinstruments zur Folge hat. Solche Aktivierungsschalter können in Form von Tasten an einem Elektrochirurgieinstrument selbst oder aber auch in Form von einen oder mehreren Fußschaltern bereitgestellt sein. Typischerweise hängt es von der Einstellung des Generators oder davon ab, wie ein jeweiliger Aktivierungsschalter oder ein jeweiliges Elektrochirurgieinstrument an den Generator angeschlossen sind, welches Elektrochirurgieinstrument oder welcher Betriebsmodus beim Betätigen eines jeweiligen Aktivierungsschalters tatsächlich aktiviert wird. Ein derartiges Elektrochirurgiesystem erlaubt es daher, die Zuordnung eines jeweiligen Aktivierungsschalters zu einem jeweiligen Elektrochirurgieinstrument oder Betriebsmodus so zu wählen, wie es für einen jeweiligen Fall oder einen jeweiligen behandelnden Arzt geeignet ist.

Aus DE 10 2009 018 107 A1 und aus GB 2 450 679 A ist jeweils ein Elektrochirurgiesystem mit einem Generator sowie einem mit dem Generator verbundenen Aktivierungsschalter bekannt, wobei der Generator zwei Betriebsmodi für ein Elektrochirurgieinstrument zur Verfügung stellen kann oder zwei Elektrochirurgieinstrumente ansteuern kann und wobei die Aktivierung oder Ansteuerung über den Aktivierungsschalter erfolgt.

In DE 20 2011 000 441 U1 ist ein Fußschalter beschrieben, der über Annäherungssensoren verfügt, die bei Annäherung des Fußes ein optisches oder akustisches Signal abgeben. Der Erfindung liegt die Aufgabe zugrunde, ein Elektrochirurgiesystem der beschriebenen Art dahingehend weiterzubilden, dass es sicherer zu bedienen ist.

Erfindungsgemäß wird die Aufgabe durch ein Elektrochirurgiesystem gelöst, welches einen Generator aufweist, mit dem wenigstens zwei Aktivierungsschalter und wenigstens ein Elektrochirurgieinstrument verbunden ist. Der Generator ist dazu ausgebildet, entweder wenigstens zwei Betriebsmodi für das erste Elektrochirurgieinstrument bereitzustellen oder zusätzlich ein zweites Elektrochirurgiesystem anzusteuern oder beides, beispielsweise eines von zwei Elektrochirurgieinstrumenten in einem von zwei Betriebsmodi anzusteuern.

Die Aktivierungsschalter sind ausgebildet, auf eine Betätigung des Aktivierungsschalters hin je ein Aktivierungssignal zu erzeugen und der Generator ist ausgebildet, auf ein Aktivierungssignal des Aktivierungsschalters hin einen der beiden Betriebsmodi oder eines der beiden Elektrochirurgieinstrumente gegebenenfalls in einem bestimmten Betriebsmodus zu aktivieren. Erfindungsgemäß ist das Elektrochirurgiesystem ausgebildet, akustisch oder optisch anzuzeigen, welcher Betriebsmodus oder welches Elektrochirurgieinstrument aktiviert ist. In diesem Zusammenhang sind die Aktivierungsschalter dazu ausgebildet, bei seiner Betätigung zunächst nur ein Voraktivierungssignal, aber noch kein Aktivierungssignal zu erzeugen, sodass nicht sofort ein Aktivieren eines jeweiligen Elektrochirurgieinstruments oder eines jeweiligen Betriebsmodus erfolgt. Vielmehr ist das Elektrochirurgiesystem ausgebildet, auf ein Voraktivierungssignal des Aktivierungsschalters hin, denjenigen Betriebsmodus oder dasjenige Elektrochirurgieinstrument anzuzeigen, der oder das aktiviert wird, wenn der Aktivierungsschalter bei weitergehender Betätigung ein entsprechendes Aktivierungssignal erzeugt. Der Generator ist ausgebildet, wenigstens zwei Voraktivierungssignale gleichzeitig zu empfangen und auf Konsistenz zu prüfen und im Falle einer fehlen- den Konsistenz ein Warnsignal auszulösen.

Hierzu ist der Generator vorzugsweise ausgebildet, Voraktivierungs- und Aktivierungssignale zu empfangen und auf ein empfangenes Voraktivierungssignal hin eine optische oder akustische Signalisierung auszulösen, die für den Benutzer wahrnehmbar ist und ihm anzeigt, welches Elektrochirurgieinstrument oder welcher Betriebsmodus voraktiviert ist. Die optische oder akustische Signalisierung ist also ein optisches oder akustisches Anzeigen eines jeweiligen voraktivierten Elektrochirurgieinstruments oder eines voraktivierten Betriebsmodus.

Mit einem erfindungsgemäßen Elektrochirurgiesystem wird somit ein jeweiliges Elektrochirurgieinstrument oder ein jeweiliger Betriebsmodus nicht sofort aktiviert, sofern der Aktivierungsschalter betätigt wird. Vielmehr kann der Aktivierungsschalter ein Voraktivierungssignal erzeugen, das es erlaubt, bereits vor Aktivierung eines jeweiligen Betriebsmodus oder Elektrochirurgieinstruments anzuzeigen, welches Elektrochirurgieinstrument oder welcher Betriebsmodus aktiviert werden wird, wenn der Aktivierungsschalter so betätigt wird, dass er das Aktivierungssignal erzeugt.

Beispielsweise kann der Aktivierungsschalter einen Näherungssensor aufweisen, der darauf anspricht, wenn beispielsweise ein Fuß oder eine Hand sich dem Aktivierungsschalter nähert. Ein solcher Näherungssensor kann dann das Voraktivierungssignal auslösen, das anzeigt, welches Elektrochirurgieinstrument oder welcher Betriebsmodus aktiviert wird, wenn der Aktivierungsschalter tatsächlich betätigt wird, beispielsweise mechanisch durch entsprechend weites Durchdrücken des Aktivierungsschalters.

Der Aktivierungsschalter ist vorzugsweise ein Fußschalter vorzugsweise mit einem Pedal, das mit einem Fuß zu betätigen ist.

Das Elektrochirurgiesystem erlaubt es die Anzahl der anschließbaren Aktivierungsschalter, beispielsweise Fußschalter und Fußschalterkombinationen zu steigern, ohne dass dies zur Beeinträchtigung der Sicherheit führt, weil ein Arzt bei dem erfindungsgemäßen Elektrochirurgiesystem immer bereits vor Aktivieren eines Instruments leicht wahrnehmbar signalisiert bekommt, welches Elektrochirurgieinstrument und welchen Betriebszustand er im Begriff ist zu aktivieren. So können beispielsweise an einen Generator bis zu drei Fußschalter angeschlossen und unterschiedlichen Instrumenten mit unterschiedlichen Funktionen zugeordnet werden. Beispielsweise können bei einem Generator drei Fußschalter mit bis zu drei Tastern zu sechs unterschiedlichen HF-Ausgängen (Sockets) des Generators bzw. daran angeschlossenen Instrumenten zugeordnet werden. Diese Zuordnung kann vorzugsweise auch an einer Anzeigeeinheit des Generators eingestellt und geprüft werden.

Die Erfindung schließt die Beobachtung ein, dass ein operierender Arzt in der Praxis ein Instrument über Handschalter oder über Fußschalter bedient. Die Einstellungen des Betriebsmodus und die Zuordnung von Fußschaltern zu einer HF-Ausgangsbuchse und damit dem dort angeschlossenen Instrument werden in der Regel durch eine Schwester am Generator selbst durchgeführt. Die Zuordnung welcher Fußschalter zu welchem Instrument und mit welcher Funktion gehört (Koagulieren, Schneiden oder Combined Mode für Ultraschallinstrumente) ist zwar auch über das Display am Generator sichtbar, damit allein jedoch nicht direkt für den operierenden Arzt erkennbar. Daraus könnten sich potentielle Handhabungsfehler durch Aktivierung von falschen Instrumenten oder einem falschen HF-Modus (Schneiden oder Koagulieren) ergeben. Zusätzlich ist es denkbar, dass bei Fußschaltern mit drei Pedalen durch Verrutschen oder durch falsches "Erfühlen" des Pedals der falsche Betriebsmodus aktiviert wird. In der Regel befinden sich die Fußschalter (und deren farbliche Kodierung) nicht im direkten Sichtfeld des operierenden Arztes. Hier schafft das Voraktivierungssignal und eine für den Arzt auch im Betrieb sichtbare oder hörbare Anzeige Abhilfe. Mit dem Elektrochirurgiesystem wird eine Möglichkeit geschaffen, dem Arzt über eine optische Anzeige am Handinstrument oder akustische Rückmeldung am Generator anzuzeigen, welcher Betriebsmodus auf welchen Instrument auf welchen Fußpedal zugeordnet ist. Dadurch ist der Arzt in der Lage ohne den Blick auf den Generator zu wenden, festzustellen, welches Instrument zu welchem Betriebsmodus auf welchem Pedal liegt.

Das Elektrochirurgiesystem bietet zwei Aspekte, die zur Lösung beitragen:
- Frühzeitiges Erkennen des Aktivierungswunsches (Vor-Aktivierung)
- Optische oder akustische Anzeige, welcher Betriebsmodus auf eine Vor-Aktivierung folgen wird.

Um ein frühzeitige Erkennung des Aktivierungswunsches zu ermöglichen, ist es vorgesehen, den Fußschalter mit einer zusätzlichen Funktion auszustatten: Diese ermöglicht es beispielweise, vor der Aktivierung - also vor dem vollständigen Herunterdrücken eines Pedals des Fußschalters - mittels einer Fußbewegung auf dem zu testenden Pedal ein Voraktivierungssignal auszulösen, das konfiguriert ist, eine Funktion im Generator auszulösen, die beispielsweise eine Leuchtanzeige auf den zugeordneten Elektrochirurgieinstrument bewirkt oder ein akustisches Signal vom Generator auslöst, das anzeigt, welcher Betriebsmodus mit dem vorbetätigten Pedal verknüpft ist.

Technisch kann eine Voraktivierung, d.h. ein Auslösen eines Voraktivierungssignals, beispielsweise durch einen Näherungssensor bewirkt werden. Bewegt der Arzt den Fuß auf oder in die Nähe eines Pedals, wird dies durch den Näherungssensor erkannt und ein entsprechendes Voraktivierungssignal ausgelöst und an den Generator weitergeleitet.

Eine weitere Möglichkeit ist ein zweistufiger Aktivierungsschalter, der durch Antippen einen ersten Kontakt aktiviert und damit ein Voraktivierungssignal auslöst und erst beim Durchdrücken des Aktivierungsschalters das eigentliche Aktivierungssignal und damit die Aktivierung auslöst.

Nachdem der Generator über die neue Aktivierungsschalterfunktion den Aktivierungswunsch erkannt hat, wird anhand der Fußschalter-zu-Anschluss-Zuordnung über die Software im Generator erkannt, zu welchem Elektrochirurgieinstrument und zu welchem Betriebsmodus dieses Pedal gehört. Anschließend wird eine Signalisierung vom Generator ausgelöst.

Vorzugsweise ist die Signalisierung optisch oder akustisch oder eine Kombination von beidem.

Zum Zwecke einer optischen Signalisierung kann in dem Elektrochirurgieinstrument ein Leuchtmittel, z.B. eine LED vorgesehen sein, das von dem Generator angesteuert werden kann und aufleuchtet, wenn das entsprechende Elektrochirurgieinstrument voraktiviert ist. Das Leuchtmittel kann vorzugsweise in verschiedenen Farben leuchten und entsprechend vom dem Generator angesteuert werden, je nachdem welcher Betriebsmodus voraktiviert ist. Das Leuchten des Leuchtmittels zeigt dem Arzt, welches Elektrochirurgieinstrument voraktiviert ist und die Farbe zeigt dem Arzt, welcher Betriebsmodus voraktiviert ist.

Im Instrument kann ein optischer Lichtleiter (z.B. POF) vorgesehen sein, der mit einem Leuchtmittel im Generator verbunden ist und das von dem Generator zum Leuchten gebracht wird. Auch hier kann durch die Farbe signalisiert werden, welcher Betriebsmodus voraktiviert ist. Ein Lichtleiter in der Zuleitung zum Elektrochirurgieinstrument macht somit ein Leuchtmittel in dem Elektrochirurgieinstrument überflüssig, so dass das Elektrochirurgieinstrument selbst günstiger sein kann. Dies ist insbesondere bei Einmal-Instrumenten von Vorteil.

Die optische Signalisierung ermöglicht ein direktes Feedback beim Arzt. Somit kann der Arzt durch "Probieren" am Fußschalter sehr intuitiv feststellen, ob das aktuelle Instrument mit dem aktuellen Pedal gesteuert werden kann.

Eine akustische Signalisierung kann per Sprachausgabe oder mit verschiedenen Tonfolgen zum Anzeigen des voraktivierten Elektrochirurgieinstruments und des voraktivierten Betriebsmodus erfolgen. Dies kann zusätzlich oder alternativ zu einer optischen Signalisierung geschehen. Insbesondere eine Unterscheidung zwischen Koagulation, Schneiden oder dem kombinierten Betriebsmodus ist mit einer akustischen Signalisierung sehr einfach zu realisieren. So kann beispielsweise die Signalisierung des voraktivierten Betriebsmodus akustisch erfolgen und die Anzeige des voraktivierten Elektrochirurgieinstruments kann optisch durch Leuchten an dem Elektrochirurgieinstrument erfolgen.

Erfindungsgemäß ist der Generator ausgebildet, wenigstens zwei Voraktivierungssignale gleichzeitig zu empfangen und auf Konsistenz zu prüfen. Beispielsweise kann in einem Handstück eines Elektrochirurgieinstruments ein Näherungssensor angeordnet sein, der ein Voraktivierungssignal erzeugt, wenn der Arzt das Elektrochirurgieinstrument in die Hand nimmt. Auch ein an dem Generator angeschlossener Fußschalter kann ausgebildet sein, ein Voraktivierungssignal zu erzeugen. Falls der Fußschalter, dem sich der Fuß des Arztes nähert, der das Elektrochirurgieinstrument in der Hand hält, diesem Fußschalter nicht zugeordnet ist, kann der Generator ein Warnsignal erzeugen.

Die Erfindung soll nun von Ausführungsbeispielen mit Bezug auf die Figuren näher erläutert werden. Von den Figuren zeigt:
- Figur 1:: ein Elektrochirurgiesystem 10 mit einem Generator und daran angeschlossenem Fußschalter 14 sowie angeschlossenen Elektrochirurgieinstrumenten 16;
- Figur 2:: eine schematische Skizze eines Fußschalters 14 für das Elektrochirurgiesystem 10 aus Figur 1;
- Figur 3:: eine schematische Skizze eines alternativen Fußschalters 14 für das Elektrochirurgiesystem 10 aus Figur 1; und
- Figur 4:: ein für das Elektrochirurgiesystem 10 aus Figur 1 geeignetes Elektrochirurgieinstrument 16 in zwei verschiedenen Anzeigezuständen.

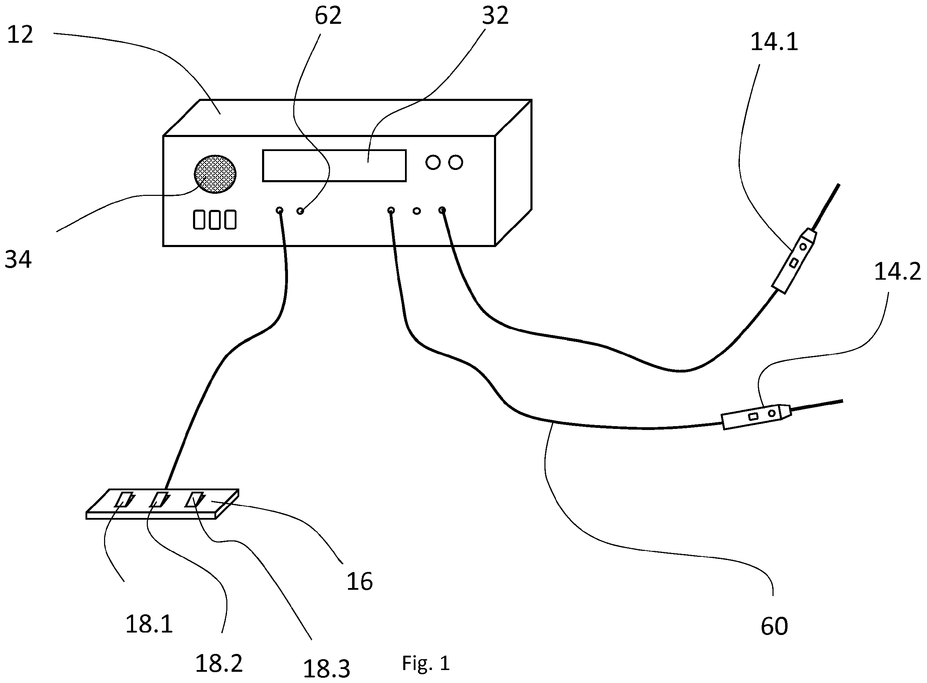

Figur 1 zeigt ein Elektrochirurgiesystem 10 mit einem Generator 12, an den zwei Elektrochirurgieinstrumente 14.1 und 14.2 angeschlossen sind. Außerdem ist an den Generator 10 eine Fußschaltereinheit 16 mit drei Fußschaltern 18.1, 18.2, 18.3 angeschlossen. Jedes Pedal 18.1, 18.2, 18.3 bildet einen Fußschalter, mit dem ein Elektrochirurgieinstrument oder ein Betriebsmodus aktiviert werden kann.

Die Elektrochirurgieinstrumente 14.1 und 14.2 sind über Zuleitungen 60 an entsprechende Generatoranschlüsse 62 des Generators 10 angeschlossen.

Die in Figur 1 skizzenhaft dargestellten Elektrochirurgieinstrumente 14.1 und 14.2 weisen jeweils eine Elektrodensonde 20 auf, die mit einem Handstück 22 fest verbunden ist. Die Elektrodensonde 20 ist von einer steifen Nadel gebildet, die zum Einstechen in Körpergewebe ausgebildet ist. Auf der Elektrodensonde 20 sind zwei Elektrodenpole angeordnet (nicht dargestellt), sodass die Elektrodensonde 20 eine bipolare Elektrodensonde ist, über deren zwei Elektrodenpole hochfrequente elektrischer Strom an umgebendes Gewebe abgegeben werden kann.

Der hochfrequente elektrische Strom wird im Betriebsfall vom Generator 12 bereitgestellt. Der vom Generator 12 bereitgestellte, hochfrequente elektrische Strom kann in verschiedener Form, beispielsweise mit verschiedenen Spannungen, Frequenzen oder Pulsformen bereitgestellt werden, um auf diese Weise verschiedene Betriebsmodi zu ermöglichen, in denen das Elektrochirurgieinstrument 14 betrieben werden kann.

Das Aktivieren eines Elektrochirurgieinstruments 14.1 oder 14.2 in einem jeweiligen Betriebsmodus erfolgt mithilfe eines von einem Arzt zu bedienenden Aktivierungsschalters.

Ein solcher Aktivierungsschalter kann von einem der Fußschalter 18.1, 18.2 oder 18.3 der Fußschaltereinheit 16 gebildet sein. Alternativ kann am Handstück 22 des Elektrochirurgieinstruments 14 ein Handschalter 24 als Aktivierungsschalter vorgesehen sein.

Welcher Betriebsmodus ausgelöst wird, wenn der Handschalter 24 eines Elektrochirurgieinstruments 14.1 oder 14.2 betätigt wird, kann in dem Generator 12 eingestellt sein.

Gleichermaßen kann in dem Generator 12 eingestellt sein, was die Betätigung eines der Fußschalter 18.1, 18.2 oder 18.3 bewirkt. Beispielsweise können alle drei Fußschalter 18.1, 18.2, 18.3 der Fußschaltereinheit 16 demselben Elektrochirurgieinstrument, beispielsweise dem Elektrochirurgieinstrument 14.2 zugeordnet sein, wobei jeder der Fußschalter 18.1, 18.2 und 18.3 einen anderen Betriebsmodus für das Elektrochirurgieinstrument 14.2 aktiviert.

Das Zuordnen von Aktivierungsschaltern 18.1, 18.2, 18.3, 24.1 und 24.2 zu einzelnen Elektrochirurgieinstrumenten und Betriebsmodi kann am Generator 12 selbst mithilfe von dort angebrachten Schaltern 30.1, 30.2 und 30.3 erfolgen. Um den Vorgang der Zuordnung zu unterstützen, weist der Generator 12 außerdem eine Anzeigeeinheit 32 auf, auf der die Zuordnung der Elektrochirurgieinstrumente und der Betriebsmodi zu den verschiedenen Schaltern angezeigt werden kann.

Der Generator 12 und die Anzeigeeinheit 32 befinden sich jedoch typischerweise im Betriebsfall nicht im Blickbereich eines behandelnden Arztes. Der Arzt müsste somit seinen Blick vom Operationsgebiet abwenden und die Anzeigeeinheit 32 des Generators 12 betrachten, wenn er sich vergewissern will, welches Elektrochirurgieinstrument er in welchem Betriebsmodus aktiviert hat, wenn er einen bestimmten Aktivierungsschalter 18.1, 18.2, 18.3, 24.1 oder 24.2 aktiviert hat. Letzteres kann der Operation abträglich sein und gegebenenfalls zu Fehlern führen, falls der Arzt versehentlich ein falsches Elektrochirurgieinstrument oder einen falschen Betriebsmodus aktiviert.

Daher weist jedes der Elektrochirurgieinstrumente 14.1 und 14.2 vorzugsweise eine optische Betriebsanzeige, beispielsweise in Form eines Leuchtmittels 26 oder eines illuminierten Abschnitts 28 des jeweiligen Handstücks 22 auf. Vorzugsweise kann das jeweilige Leuchtmittel 26 oder der jeweilige illuminierte Abschnitt 28 des Handstücks 22 in verschiedenen Farben leuchten, um auf diese Weise verschiedene Betriebsmodi anzuzeigen.

Beispielsweise kann das Leuchtmittel 26 oder der illuminierte Abschnitt 28 des Handstücks 22 grün leuchten, wenn das Elektrochirurgieinstrument 14.2 in einem Koagulationsmodus aktiviert ist und das Leuchtmittel 26 oder der illuminierte Abschnitt 28 kann rot leuchten, wenn das Elektrochirurgieinstrument 14.2 in einem Schneidmodus aktiviert wird.

Alternativ oder zusätzlich ist außerdem am Generator ein Schallgeber 34, beispielsweise ein Lautsprecher vorgesehen, der unterschiedliche Schallsignale ausgeben kann, um auf diese Weise anzuzeigen, welches Elektrochirurgieinstrument aktiviert ist und in welchem Betriebsmodus das Elektrochirurgieinstrument aktiviert ist. Als Schallsignal kommen beispielsweise Tonfolgen infrage, die unterschiedliche Elektrochirurgieinstrumente 14.1 oder 14.2 und unterschiedliche Betriebsmodi kennzeichnen. Zusätzlich oder alternativ kann der Generator 12 auch ausgebildet sein, über den Schallgeber 34 Klartextansagen auszugeben, beispielsweise "Instrument eins, Koagulationsmodus bipolar", wenn das erste Elektrochirurgieinstrument 14.1 in einem bipolaren Koagulationsmodus als Betriebsmodus aktiviert wird.

Die Fußschalter 18.1, 18.2 und 18.3 sowie die Handstücke 22.1 und 22. 2 sind so ausgebildet, dass sie vor einer ein Elektrochirurgieinstrument oder einen Betriebsmodus aktivierenden Betätigung ein Voraktivierungssignal auslösen, welches drahtlos oder drahtgebunden zu dem Elektrochirurgiegenerator 12 übermittelt wird und dort bewirkt, dass der Elektrochirurgiegenerator 12 eine entsprechende optische oder akustische Signalisierung im zuvor erwähnten Sinne auslöst. Beispielsweise kann jeder Fußschalter 18.1, 18.2 oder 18.3 oder jedes Handstück 22.1 und 22.2 mit einem Näherungssensor ausgestattet sein, der ein Signal abgibt, wenn sich ein Fuß einem jeweiligen der Fußschalter 18.1, 18.2 oder 18.3 nähert oder wenn der Chirurg das Handstück 22.1 oder 22.2 des Elektrochirurgieinstruments 14.1 oder 14.2 greift.

Alternativ oder zusätzlich ist es auch möglich, dass die Fußschalter 18.1, 18.2 und 18.3 oder die Handschalter 24.1 und 24.2 als zweistufige Schalter ausgebildet sind, die halb und vollständig gedrückt werden können, um bei halbem Druck auf den jeweiligen Fuß- oder Handschalter das Voraktivierungssignal auszulösen und bei vollständigem Druck auf den Fuß- oder Handschalter das eigentliche Aktivierungssignal auszulösen, das ein bestimmtes Elektrochirurgieinstrument in einem jeweiligen bestimmten Betriebsmodus aktiviert. Derartige zweistufige Fuß- oder Handschalter weisen vorzugsweise einen ersten Druckpunkt auf, wenn der jeweilige Fuß- oder Handschalter soweit betätigt ist, dass er das Voraktivierungssignal abgibt, um dann das eigentliche Aktivierungssignal auszulösen ist ein etwas kräftigerer Druck auf den jeweiligen Fuß- oder Handschalter erforderlich, um den ersten Druckpunkt zu überwinden und den Fuß- oder Handschalter vollständig so zu betätigen, dass er ein Aktivierungssignal ausgibt, welches drahtgebunden oder drahtlos an den Elektrochirurgiegenerator 12 übermittelt wird.

Der Elektrochirurgiegenerator 12 ist ausgebildet, seitens einzelner Fuß- oder Handschalter empfangene Voraktivierungs- und Aktivierungssignale zu empfangen und auszuwerten.

Insbesondere ist der Elektrochirurgiegenerator 12 ausgebildet, Voraktivierungssignale dahingehend auszuwerten, dass er bei Empfang eines jeweiligen Voraktivierungssignals eine optische und/oder akustische Anzeige auslöst, die einem Arzt anzeigt, welches Elektrochirurgieinstrument und welcher Betriebsmodus dem jeweils vorbetätigten Fuß- oder Handschalter zugeordnet ist. Der Arzt kann daraufhin prüfen, ob das angezeigte Elektrochirurgieinstrument und der angezeigte Betriebsmodus das gewünschte Elektrochirurgieinstrument im gewünschten Betriebsmodus ist und dann den jeweiligen Fuß-¨ oder Handschalter entsprechend weiter durchzudrücken, um so tatsächlich die Abgabe der gewünschten Energieform (die den Betriebsmodus bestimmt) über das gewünschte Elektrochirurgieinstrument auslösen.

Weiterhin kann der Elektrochirurgiegenerator 12 ausgebildet sein, auf Basis von Voraktivierungssignalen eine Plausibilitätsprüfung durchzuführen. Falls der Elektrochirurgiegenerator 12 beispielsweise ein Voraktivierungssignal von dem ersten Elektrochirurgieinstrument 14.2 empfängt, weil der Arzt das Handstück 22.1 in die Hand genommen und damit einen in dem Handstück 22.1 angeordneten Näherungssensor ausgelöst hat, während der Arzt gleichzeitig beispielsweise den zweiten Fußschalter 18.2 halb betätigt hat, um auf diese Weise ein Voraktivierungssignal vom zweiten Fußschalter 18.2 auszulösen, kann eine Steuerung des Elektrochirurgiegenerators 12 ermitteln, ob das erste Elektrochirurgieinstrument 14.1 tatsächlich dem Fußschalter 18.2 zugeordnet ist. Falls Letzteres nicht der Fall ist, kann die Steuerung des Elektrochirurgiegenerators 12 ein Warnsignal auslösen, das dem Arzt anzeigt, dass das Elektrochirurgieinstrument, das er in die Hand genommen hat, nicht dem Fußschalter 18.2 zugeordnet ist, den er bereits voraktiviert hat.

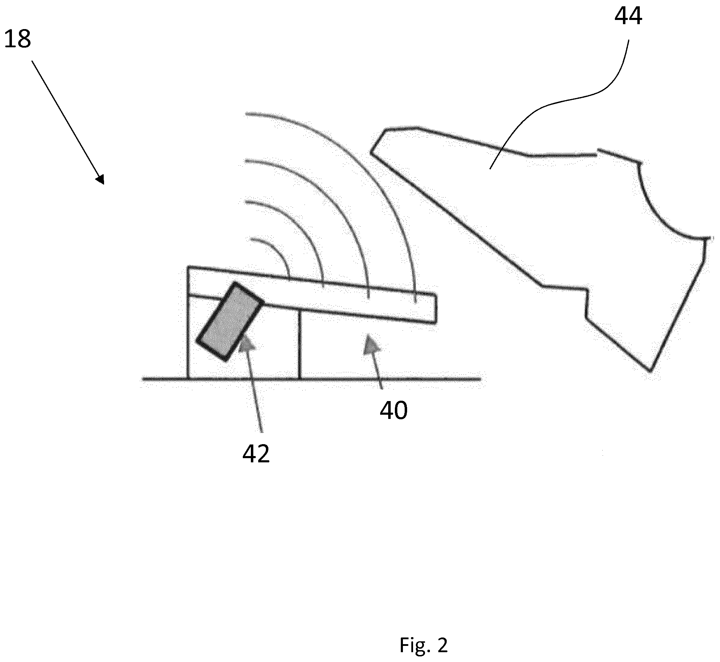

Figur 2 zeigt beispielhaft einen Fußschalter 18', der ein Pedal 40 und einen Näherungssensor 42 aufweist. Der Näherungssensor 42 ist so angeordnet und ausgebildet, dass er auf ein Annähern eines Fußes 44 anspricht, sobald sich der Fuß 44 ganz nahe oder auf dem Pedal 40 befindet. Sobald sich ein Fuß 44 dem Näherungssensor 42 nahe genug nähert, löst der Fußschalter 18' das Voraktivierungssignal aus und übermittelt dieses drahtlos oder drahtgebunden an den Generator 12. Erst wenn der Fuß 44 das Pedal 40 durchdrückt, löst der Fußschalter 18' das eigentliche Aktivierungssignal aus, das den Generator 12 dazu veranlasst, den mit dem Fußschalter 18 verknüpften Betriebsmodus für ein jeweiliges Elektrochirurgieinstrument auszulösen.

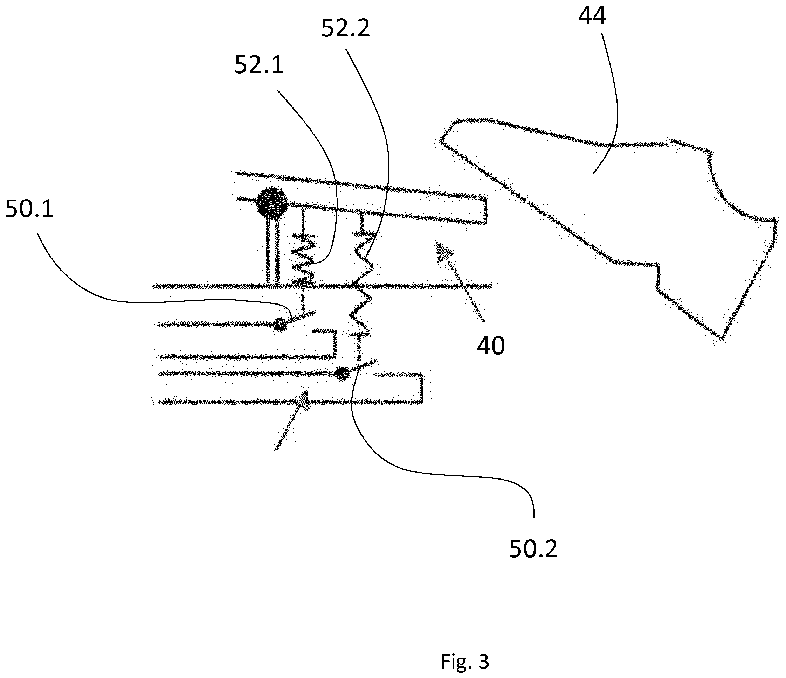

Figur 3 zeigt einen alternativen Fußschalter 18", der keinen Näherungssensor aufweist, sondern als zweistufige Taste ausgebildet ist.

Wie Figur 3 zu entnehmen ist, weist der Fußschalter 18" zwei Schaltkontakte 50.1 und 50.2 auf, die jeweils über Druckfedern 52.1 und 52.2 mit einem Pedal 40" des Fußschalters 18" verbunden sind. Die Druckfedern 52.1 und 52.2 sind so eingestellt, dass sie verschiedene Auslösekräfte bewirken, bei denen der Schaltkontakt 50.1 oder 50.2 ausgelöst wird. Auf diese Weise ist es möglich, das Pedal 40" zunächst bis zu einem ersten Druckpunkt zu betätigen, bei denen ein erster der Schaltkontakte 50.1 oder 50.2, beispielsweise der Schaltkontakt 50.1, geschlossen wird. Das Schließen des ersten Schaltkontakts 50.1 bewirkt die Ausgabe des Voraktivierungssignals. Wird das Pedal 40 über den ersten Druckpunkt hinaus mit entsprechend größerer Fußkraft weiter betätigt, schließt auch der zweite Schaltkontakt 50.2 und löst das dem Fußschalter 18" zugeordnete Aktivierungssignal aus.

Ähnlich der in den Figuren 2 und 3 dargestellten Ausführungsbeispiele für einen Fußschalter 18' bzw. 18" können auch die Handschalter 24.1 und 24.2 an einem jeweiligen Elektrochirurgieinstrument 14.1 oder 14.2 ausgebildet sein.

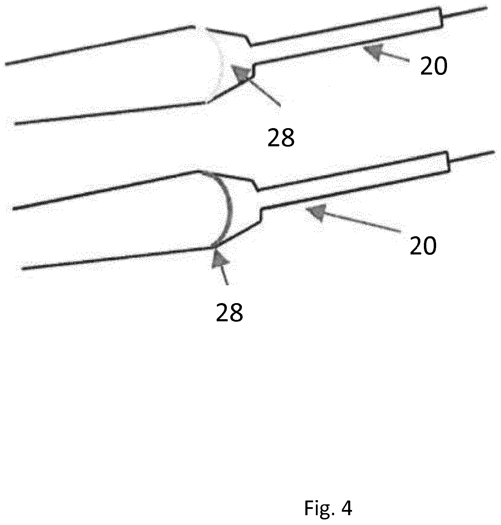

In Figur 4 ist angedeutet, wie mittels einer optischen Anzeige am Elektrochirurgieinstrument 14.1 oder 14.2 selbst angezeigt werden kann, welches Elektrochirurgieinstrument und welcher Betriebsmodus aktiviert ist. Dazu weist das Elektrochirurgieinstrument 14 ein Leuchtmittel auf, das beispielsweise in wenigstens zwei verschiedenen Farben leuchten kann. Wird nun das Elektrochirurgieinstrument 14 in einem bestimmten Betriebsmodus aktiviert, bewirkt dies, dass das Leuchtmittel 26 an dem Elektrochirurgieinstrument 14 aktiviert wird, und zwar in der Farbe, die dem Betriebsmodus entspricht, der durch den Fuß- oder Handschalter ausgelöst wird, dem dieser Betriebsmodus auf diesem Elektrochirurgieinstrument zugordnet ist.

### Bezugszeichenliste

- 10: Elektrochirurgiesystem
- 12: Generator
- 14, 14.1, 14.2: Elektrochirurgieinstrument
- 16: Fußschaltereinheit
- 18, 18.1, 18.2, 18.3, 18', 18": Fußschalter
- 20: Elektrodensonde
- 22, 22.1, 22.2: Handstück
- 24, 24.1, 24.2: Handschalter
- 26: Leuchtmittel
- 28: illuminierter Abschnitt
- 30.1, 30.2 und 30.3: Schalter
- 32: Anzeigeeinheit
- 34: Schallgeber
- 40, 40": Pedal
- 42: Näherungssensor
- 44: Fuß
- 50.1, 50.2: Schaltkontakte
- 52.1, 52.2: Druckfedern
- 60: Zuleitung
- 62: Generatoranschluss

## Patentansprüche

1. Elektrochirurgiesystem (10), welches einen Generator (12) und wenigstens zwei mit dem Generator (12) verbundene Aktivierungsschalter (18; 24) und wenigstes ein mit dem Generator (12) verbundenes Elektrochirurgieinstrument (14.1, 14.2) umfasst, wobei der Generator dazu ausgebildet ist, entweder wenigstens zwei Betriebsmodi für das erste Elektrochirurgieinstrument (14.1, 14.2) bereitzustellen oder zusätzlich ein zweites Elektrochirurgieinstrument (14.1, 14.2) anzusteuern oder beides, wobei die Aktivierungsschalter (18, 24) ausgebildet sind, auf eine Betätigung des Aktivierungsschalters (18, 24) hin je ein Aktivierungssignal zu erzeugen und
wobei der Generator weiterhin ausgebildet ist, auf ein Aktivierungssignal des Aktivierungsschalters (18, 24) hin einen der beiden Betriebsmodi oder eines der beiden Elektrochirurgieinstrumente (14.1, 14.2) zu aktivieren,
wobei das Elektrochirurgiesystem (10) ausgebildet ist, akustisch oder optisch anzuzeigen, welcher Betriebsmodus aktiviert oder welches Elektrochirurgieinstrument (14.1, 14.2) aktiviert ist,
**dadurch gekennzeichnet, dass**
die Aktivierungsschalter (18, 24) ausgebildet sind, bei Betätigung zunächst ein Voraktivierungssignal, aber noch kein Aktivierungssignal zu erzeugen,
wobei das Elektrochirurgiesystem (10) ausgebildet ist, auf ein Voraktivierungssignal des Aktivierungsschalters (18, 24) hin denjenigen Betriebsmodus oder dasjenige Elektrochirurgieinstrument (14.1, 14.2) anzuzeigen, der oder das aktiviert wird, wenn der Aktivierungsschalter (18, 24) bei weitergehender Betätigung ein Aktivierungssignal erzeugt,
wobei der Generator (12) ausgebildet ist, wenigstens zwei Voraktivierungssignale gleichzeitig zu empfangen und auf Konsistenz zu prüfen und im Falle einer fehlenden Konsistenz ein Warnsignal auszulösen.

2. Elektrochirurgiesystem gemäß Anspruch 1, **dadurch gekennzeichnet, dass** der Aktivierungsschalter ein Fußschalter (18) ist.

3. Elektrochirurgiesystem gemäß Anspruch 1 oder 2, **dadurch gekennzeichnet, dass** der Aktivierungsschalter ein zweistufiger Schalter ist, der ausgebildet, bei einer Betätigung der ersten Schaltstufe ein Voraktivierungssignal und bei einer Betätigung der zweiten Schaltstufe ein Aktivierungssignal zu erzeugen.

4. Elektrochirurgiesystem gemäß wenigstens einem der Ansprüche 1 bis 3, **dadurch gekennzeichnet, dass** der Aktivierungsschalter einen Näherungssensor aufweist, der angeordnet uns ausgebildet ist, eine Annäherung an den Aktivierungsschalter zu erfassen und im Falle einer erfassten Annäherung ein Voraktivierungssignal zu erzeugen.

5. Elektrochirurgiesystem gemäß wenigstens einem der Ansprüche 1 bis 4, **dadurch gekennzeichnet, dass** wenigstens ein Leuchtmittel (26) vorgesehen ist und dass der Generator (12) ausgebildet ist, mittels des Leuchtmittels (26) optisch zu signalisieren, dass ein bestimmtes Elektrochirurgieinstrument (14.11, 14.2) voraktiviert ist.

6. Elektrochirurgiesystem gemäß Anspruch 5, **dadurch gekennzeichnet, dass** das Leuchtmittel (26) so angeordnet ist, dass ein Leuchten des Leuchtmittels (26) an dem Elektrochirurgieinstrument (14.1, 14.2) wahrnehmbar, welches durch ein Voraktivierungssignal voraktiviert ist.

7. Elektrochirurgiesystem gemäß Anspruch 5 oder 6, **dadurch gekennzeichnet, dass** das Leuchtmittel (26) ausgebildet ist, in wenigstens zwei verschieden Farben zu leuchten, und der Generator (12) ausgebildet ist, das Leuchtmittel (26) derart anzusteuern, dass die Farbe des Leuchtmittels (26) anzeigt, welcher Betriebsmodus für ein entsprechend voraktiviertes Elektrochirurgieinstrument voraktiviert ist.

8. Elektrochirurgiesystem gemäß wenigstens einem der Ansprüche 5 bis 7, **dadurch gekennzeichnet, dass** das Elektrochirurgieinstrument (14.1, 14.2) mittels einer Zuleitung (60) an einen Generatoranschluss (62) des Generators (12) angeschlossen ist, wobei die Zuleitung (60) einen Lichtleiter aufweist und das Leuchtmittel derart in dem Generator (12) angeordnet ist, dass Licht von dem Leuchtmittel in den Lichtleiter eintreten kann, und dass das Elektrochirurgieinstrument (14.1, 14.2) einen illuminierten Abschnitt (28) aufweist, der leuchtet, wenn Licht mittels des Lichtleiters in das Elektrochirurgieinstrument (14.1, 14.2) geleitet wird.

9. Elektrochirurgiesystem gemäß wenigstens einem der Ansprüche 1 bis 8, **dadurch gekennzeichnet, dass** der Generator (12) einen Schallgeber (36) aufweist oder mit einem solchen verbunden ist, wobei der Generator ausgebildet ist, einen voraktivierten Betriebsmodus akustisch durch ein entsprechendes, dem jeweiligen Betriebsmodus zugeordnetes Tonsignal akustisch zu signalisieren.

10. Elektrochirurgiesystem gemäß wenigstens einem der Ansprüche 1 bis 9, **dadurch gekennzeichnet, dass** der Generator (12) mehrere Generatoranschlüsse (62) aufweist, an die jeweils ein Elektrochirurgieinstrument (14.1, 14.2) oder ein Aktivierungsschalter (18, 24) angeschlossen werden kann, wobei der Generator (12) ausgebildet ist, einen an den Generator (12) angeschlossenen Aktivierungsschalter (18, 24 jeweils einem Elektrochirurgieinstrument (14.1, 14.2) oder einem Betriebsmodus oder einer Kombination aus Elektrochirurgieinstrument (14.1, 14.2) und Betriebsmodus zuzuordnen.

## Claims

1. An electrosurgical system (10) comprising a generator (12), at least two activation switches (18; 24) connected to the generator (12), and at least one electrosurgical instrument (14.1, 14.2) connected to the generator (12),
wherein the generator is configured to either provide at least two operating modes for the first electrosurgical instrument (14.1, 14.2) or to additionally control a second electrosurgical instrument (14.1, 14.2), or both,
wherein the activation switches (18, 24) are configured to each generate an activation signal in response to actuation of the activation switch (18, 24), and
wherein the generator is further configured to activate one of the two operating modes or one of the two electrosurgical instruments (14.1, 14.2) in response to an activation signal from the activation switch (18, 24),
wherein the electrosurgical system (10) is configured to indicate, acoustically or visually, which operating mode or which electrosurgical instrument (14.1, 14.2) is activated,
**characterized in that** the activation switches (18, 24) are configured to generate, upon actuation, first a pre-activation signal but not yet an activation signal,
wherein the electrosurgical system (10) is configured to indicate, in response to a pre-activation signal from the activation switch (18, 24), the operating mode or the electrosurgical instrument (14.1, 14.2) that will be activated when the activation switch (18, 24) generates an activation signal upon further actuation,
wherein the generator (12) is configured to receive at least two pre-activation signals simultaneously, to check the two pre-activation signals for consistency, and to trigger a warning signal in the event of a lack of consistency.

2. An electrosurgical system according to claim 1, **characterized in that** the activation switch is a foot switch (18).

3. An electrosurgical system according to claim 1 or 2, **characterized in that** the activation switch is a two-stage switch configured to generate a pre-activation signal upon actuation of the first switching stage and an activation signal upon actuation of the second switching stage.

4. An electrosurgical system according to at least one of claims 1 through 3, **characterized in that** the activation switch comprises a proximity sensor that is arranged and configured to detect an approach to the activation switch and, upon detecting such an approach, to generate a pre-activation signal.

5. An electrosurgical system according to at least one of claims 1 through **4, characterized in that** at least one light source (26) is provided and that the generator (12) is configured to use the light source (26) to visually indicate that a specific electrosurgical instrument (14.11, 14.2) is pre-activated.

6. An electrosurgical system according to claim 5, **characterized in that** the light source (26) is arranged such that the illumination of the light source (26) is visible on the electrosurgical instrument (14.1, 14.2) that has been preactivated by a preactivation signal.

7. An electrosurgical system according to claim 5 or 6, **characterized in that** the light source (26) is configured to emit light in at least two different colors, and the generator (12) is configured to drive the light source (26) such that the color of the light source (26) indicates which operating mode is preactivated for a correspondingly preactivated electrosurgical instrument.

8. An electrosurgical system according to at least one of claims 5 through 7, **characterized in that** the electrosurgical instrument (14.1, 14.2) is connected via a supply line (60) to a generator terminal (62) of the generator (12), wherein the supply line (60) comprises an optical fiber and the light source is arranged within the generator (12) such that light from the light source can enter the optical fiber, and that the electrosurgical instrument (14.1, 14.2) comprises an illuminated section (28) that glows when light is guided into the electrosurgical instrument (14.1, 14.2) via the light guide.

9. An electrosurgical system according to at least one of claims 1 through 8, **characterized in that** the generator (12) comprises or is connected to a sound generator (36), wherein the generator is configured to acoustically signal a pre-activated operating mode by means of a corresponding tone signal associated with the respective operating mode.

10. An electrosurgical system according to at least one of claims 1 through 9, **characterized in that** the generator (12) comprises a plurality of generator terminals (62), to each of which an electrosurgical instrument (14.1, 14.2) or an activation switch (18, 24) can be connected, wherein the generator (12) is configured to assign an activation switch (18, 24) connected to the generator (12) to a respective electrosurgical instrument (14.1, 14.2) or an operating mode, or a combination of an electrosurgical instrument (14.1, 14.2) and an operating mode.

## Revendications

1. Système (10) électrochirurgical comportant un générateur (12) et au moins deux commutateurs (18 ; 24) d'activation connectés au générateur (12) et au moins un instrument (14.1, 14.2) électrochirurgical connecté au générateur (12), le générateur étant configuré de manière à fournir soit au moins deux modes de fonctionnement pour le premier instrument (14.1, 14.2) électrochirurgical, soit en plus pour commander un deuxième instrument (14.1, 14.2) électrochirurgical ou les deux, dans lequel les commutateurs (18, 24) d'activation sont configurés pour produire un signal d'activation à la suite d'un actionnement du commutateur (18, 24) d'activation et dans lequel le générateur est configuré en outre pour activer l'un des deux modes de fonctionnement ou l'un des deux instruments (14.1, 14.2) électrochirurgical à la suite d'un signal d'activation du commutateur (18, 24) d'activation,
dans lequel le système (10) électrochirurgical est configuré pour réaliser une indication acoustique ou optique, cette dernière activant le mode de fonctionnement ou activant l'instrument (14.1, 14.2) électrochirurgical,
**caractérisé en ce que** les commutateurs (18, 24) d'activation sont configurés, lors de l'actionnement, pour produire d'abord un signal de préactivation, mais pas encore un signal d'activation,
dans lequel le système (10) électrochirurgical est configuré pour réaliser une indication, à la suite d'un signal de préactivation provenant du commutateur (18, 24) d'activation, du mode de fonctionnement ou de l'instrument (14.1, 14.2) électrochirurgical, qui est activé lorsque le commutateur (18, 24) d'activation produit un signal d'activation lors d'un actionnement ultérieur,
dans lequel le générateur (12) est configuré pour recevoir au moins deux signaux de préactivation simultanément et pour vérifier la cohérence et déclencher un signal d'avertissement dans le cas d'un manque de cohérence.

2. Système électrochirurgical suivant la revendication 1, **caractérisé en ce que** le commutateur d'activation est un commutateur (18) à pédale.

3. Système électrochirurgical suivant la revendication 1 ou 2, **caractérisé en ce que** le commutateur d'activation est un commutateur à deux étages, qui est configuré pour produire un signal de préactivation lors de l'actionnement du premier étage de commutation et pour produire un signal d'activation lors de l'actionnement du deuxième étage de commutation.

4. Système électrochirurgical suivant au moins l'une des revendications 1 à 3, **caractérisé en ce que** le commutateur d'activation comporte un capteur de proximité qui est agencé et configuré pour détecter une proximité au commutateur d'activation et pour produire un signal de préactivation dans le cas d'une proximité détectée.

5. Système électrochirurgical suivant au moins l'une des revendications 1 à 4, **caractérisé en ce qu'**au moins un moyen (26) lumineux est prévu et **en ce que** le générateur (12) est configuré pour réaliser une signalisation optique par le moyen (26) lumineux du fait qu'un instrument (14.11, 14.2) électrochirurgical déterminé est préactivé.

6. Système électrochirurgical suivant la revendication 5, **caractérisé en ce que** le moyen (26) lumineux est agencé de sorte qu'une illumination du moyen (26) lumineux peut être perçue sur l'instrument (14.1, 14.2) électrochirurgical qui est préactivé par un signal de préactivation.

7. Système électrochirurgical suivant la revendication 5 ou 6, **caractérisé en ce que** le moyen (26) lumineux est réalisé pour illuminer en au moins deux couleurs différentes, et le générateur (12) est configuré pour commander le moyen (26) lumineux de sorte que la couleur du moyen (26) lumineux montre quel mode de fonctionnement est préactivé pour un instrument électrochirurgical préactivé correspondant.

8. Système électrochirurgical suivant au moins l'une des revendications 5 à 7, **caractérisé en ce que** l'instrument électrochirurgical (14.1, 14.2) est connecté au moyen d'une ligne (60) d'alimentation à une borne (62) du générateur (12), dans lequel la ligne (60) d'alimentation comporte un guide de lumière et le moyen lumineux est agencé dans le générateur (12) de sorte que de la lumière provenant du moyen lumineux peut entrer dans le guide de lumière, et **en ce que** l'instrument (14.1, 14.2) électrochirurgical comporte un tronçon (28) illuminé, qui s'illumine lorsque de la lumière est guidée au moyen du guide de lumière dans l'instrument (14.1, 14.2) électrochirurgical.

9. Système électrochirurgical suivant au moins l'une des revendications 1 à 8, **caractérisé en ce que** le générateur (12) comporte un émetteur (36) sonore ou est relié à un tel émetteur sonore, dans lequel le générateur est configuré pour signaler acoustiquement un mode de fonctionnement préactivé par l'intermédiaire d'un signal de tonalité associé à un mode de fonctionnement correspondant.

10. Système électrochirurgical suivant au moins l'une des revendications 1 à 9, **caractérisé en ce que** le générateur (12) comporte plusieurs bornes (62) de générateur, auxquelles un instrument (14.1, 14.2) électrochirurgical ou un commutateur (18, 24) d'activation peuvent être connectés, le générateur (12) étant configuré pour attribuer un commutateur (18, 24) d'activation connecté au générateur (12) à un instrument (14.1, 14.2) électrochirurgical ou à un mode de fonctionnement ou à une combinaison d'instrument (14.1, 14.2) électrochirurgical et de mode de fonctionnement.
